(19) Europäisches Patentamt
European Patent Office
Office européen des brevets

(11) EP 1 449 514 A1

(12) **EUROPÄISCHE PATENTANMELDUNG**

(43) Veröffentlichungstag:
**25.08.2004 Patentblatt 2004/35**

(51) Int Cl.7: **A61K 7/48**

(21) Anmeldenummer: **03003098.5**

(22) Anmeldetag: **13.02.2003**

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(71) Anmelder: **Beiersdorf AG**
**20245 Hamburg (DE)**

(72) Erfinder:
- **Raschke, Thomas, Dr.**
  **25421 Pinneberg (DE)**
- **Schimpf, Ralph, Dr.**
  **25474 Bönningstedt (DE)**
- **Max, Heiner, Dr.**
  **22529 Hamburg (DE)**

(54) **Hautpflegeprodukte mit Retinoiden, Ubichinonen sowie Biotin oder Carnitin**

(57) Kosmetische und/oder dermatologische Zubereitungen enthaltend

a) mindestens ein Retinoid
b) mindestens ein Ubichinon und/oder ein Derivat davon
c) mindestens einen stickstoffhaltigen Wirkstoff aus der Gruppe Biotin, Carnitin und/oder deren Derivate

neben anderen kosmetischen Hilfs-, Wirk- und Zusatzstoffen.

EP 1 449 514 A1

## Beschreibung

Die vorliegende Erfindung betrifft kosmetische und/oder dermatologische Zubereitungen enthaltend

a) mindestens ein Retinoid,
b) mindestens ein Ubichinon und/oder ein Derivat davon und
c) mindestens einen stickstoffhaltigen Wirkstoff aus der Gruppe Biotin, Carnitin und/oder deren Derivate

neben anderen kosmetischen Hilfs-, Wirk- und Zusatzstoffen und deren Verwendung.

Die Haut ist das größte Organ des Menschen. Unter ihren vielen Funktionen (beispielsweise zur Wärmeregulation und als Sinnesorgan) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (Stratum corneum), den für die Barrierefunktion bedeutenden Teil darstellt. Sie wird im Kontakt mit der Umwelt abgenutzt und befindet sich deshalb in einem ständigen Erneuerungsprozess, wobei nach außen kontinuierlich feine Schuppen abgegeben und von innen verhorntes Zell- und Lipidmaterial nachproduziert wird.

Das heute in der Fachwelt anerkannte Hautmodell von Elias *(P. M. Elias, Structure and Function of the Stratum Corneum Permeability Barrier, Drug Dev. Res.* ***13****, 1988, 97-105*) beschreibt die Hornschicht als Zwei-Komponenten-System, ähnlich einer Ziegelsteinmauer (Ziegelstein-Mörtel-Modell). In diesem Modell entsprechen die Hornzellen (Körneozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben.

Unter kosmetischer Hautpflege ist in erster Linie zu verstehen, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z. B. nach Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schläffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Die vorliegende Erfindung betrifft insbesondere Produkte zur Pflege der durch Umweltnoxen wie z. B. UV-Licht, Ozon, Zigarettenrauch gestressten Haut, sowie zur Behandlung der Folgeschäden der Lichtalterung, insbesondere der unter a) bis g) aufgeführten Phänomene.

Produkte zur Pflege der Haut sind an sich bekannt. Sie enthalten z. B. Retinoide (Vitamin A-Säure und/oder deren Derivate) bzw. Vitamin A und/oder dessen Derivate. Retinoide gehören zur Stoffklasse der monocyclischen Diterpene. Der primäre ungesättigte Alkohol Retinol, auch Vitamin A genannt, hat die folgende Struktur:

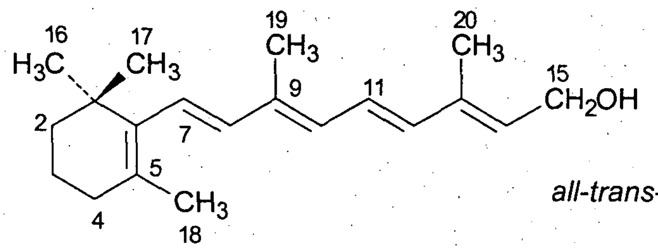

*all-trans*-Retinol, Retinol

Eine Reihe von Retinoiden wirken stark antiseboretisch (mit einer Reduktion der Talgdrüsenaktivität auf 10 Prozent des Ausgangswertes), antikeratinisierend, proliferationsanregend auf die Epidermiszellen und zellteilungshemmend bei patologisch erhöhter Zellteilungsrate. Aufgrund dieser Effekte sind solche Retinoide bei Akne wirksam. Dies gilt insbesondere für die Retinoide Tretinoin und Adapalen (G. Kindl, W. Raab, Licht und Haut, Govi-Verlag, 4. Auflage).

Retinoide sind in zahlreichen Patenten beschrieben. Die Schriften WO 98/52536, 98/14167, 98/25587 und EP 0 676 194 schildern Retinoide in verschiedenen Anwendungen. In den Schriften WO 97/31620 und WO 96/07396 werden u.a. Kombinationen aus Retinoiden und Biotin beschrieben, die jedoch nicht den Weg zur vorliegenden Erfindung weisen konnten, da Biotin hier lediglich optional als Antioxidans eingesetzt wird. Die DE 199 43 678 schließlich behandelt die Kombination aus Retinoiden und Cyclodextrinen.

Ubichinone sind eine Gruppe von Substanzen, die an ihrem Chinon-Ring n Isopren-einheiten kettenförmig gebunden haben. Ubichinone fungieren bei der biologischen, mitochondalen Oxidation als Elektronenüberträger und spielen so eine bedeutende Rolle im Energiestoffwechsel der Zellen.

Die Ubichinone sind aus der Literatur bekannt (z.B. Beyer, Walter, Lehrbuch der Organischen Chemie, S. Hirzel-Verlag, Stuttgart, 22. Auflage). Sie werden auch als Mitochinone oder Coenzyme Q bezeichnet. Die Anzahl der Isopreneinheiten in der Seitenkette wird mit n in der Bezeichnung Coenzym Q-n angegeben, worin n eine ganze Zahl bedeutet. Es sind Coenzyme von n = 0 bis n = 12 bekannt. Am häufigsten sind jedoch n=6-10.

In kosmetischen Zubereitungen finden Ubichinone seit langem Anwendung als Antioxidantien zum Schutz oxidationsempfindlicher Substanzen. So beschreibt die WO 95/26181 Ubichinone in topischen Zubereitungen. Die WO 95/26180 beschreibt die Kombination von Ubichinonen und Retinolen als Hautpflegemittel. Der Nachteil im Stande der Technik besteht hier in der schlechten Bioverfügbarkeit der Verbindungen, die meist mit der Zubereitung auf der Haut liegen bleiben.

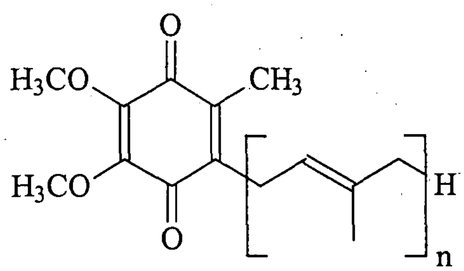

Ubichinone n = 6 - 10

Biotin (Vitamin H) gehört zu den Biowuchsstoffen, die das embryonale oder plasmatische Wachstum der Zellen regulieren, und ist ein unentbehrlicher Faktor für die Vermehrung der Hefe und anderer Mikroorganismen. Im menschlichen Organismus hat es sich als ein für die normale Funktion der Haut notwendiger Wirkstoff, Vitamin H, erwiesen (Beyer, Walter, Lehrbuch der Organischen Chemie, 22.Aufl., S. Hirzel Verlag). Biotin ist ein chirales Molekül mit drei asymmetrischen Kohlenstoffatomen und besitzt die folgende Struktur:

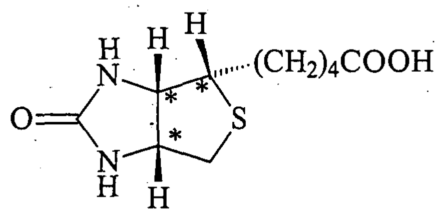

Natürliches Biotin ist rechtsdrehend und kann aus D-Mannose synthetisiert werden.
In jüngerer Zeit findet Biotin zunehmend in der Kosmetik Verwendung (z.B.: DE 4242876 und DE 19941769). In der Regel ist jedoch die Wirksamkeit des Biotins eher mäßig, da die Verbindung nur schlecht von der Haut aufgenommen wird.
Carnitin (3-Hydroxy-4-(trimethylammonio)-buttersäurebetain) ist eine Verbindung, die als Zwitterion vorliegt. Die L-Form ist in tierischen, Geweben weit verbreitet und ein charakteristischer Bestandteil der gestreiften Muskulatur (lat.: carnis = Fleisch). Es dient im Fettstoffwechsel als Überträger von Acylgruppe durch die Mitochondrienmembran hindurch.

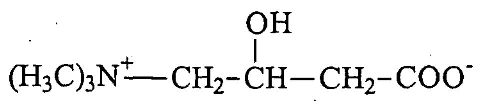

Carnitin

Es war nun die Aufgabe der vorliegenden Erfindung wirksame, die Mängel des Standes der Technik beseitigende kosmetische und/oder dermatologische Zubereitungen zu entwickeln, die für die Prophylaxe und Pflege der Altershaut, insbesondere der trockenen Altershaut bzw. der lichtgealterten Haut geeignet sind.
Vollkommen überraschend und für den Fachmann nicht vorhersehbar wird die Aufgabe gelöst durch kosmetische und/oder dermatologische Zubereitungen enthaltend

a) mindestens ein Retinoid,
b) mindestens ein Ubichinon und/oder ein Derivat davon und
c) mindestens einen stickstoffhaltigen Wirkstoff aus der Gruppe Biotin, Carnitin und/oder deren Derivate neben anderen kosmetischen Hilfs-, Wirk- und Zusatzstoffen.

Erfindungsgemäß vorteilhaft sind dabei Zubereitungen, die

a) Retinoid in einer Konzentration von 0.001 bis 1.0 Gewichts-% und besonders bevorzugt in einer Konzentration von 0.01 bis 0.2 Gewichts-%,
b) Ubichinon in einer Konzentration von 0.001 bis 1.0 Gewichts-% und besonders bevorzugt in einer Konzentration von 0.01 bis 0.3 Gewichts-% und
c) Biotin, Carnitin und/oder deren Derivate in einer Konzentration von 0.001 bis 5 Gewichts-% und besonders bevorzugt in einer Konzentration von 0.02 bis 2 Gewichts-%

jeweils bezogen auf das Gesamtgewicht der Zubereitung enthalten.
Das erfindungsgemäß bevorzugte Retenoid ist Retinol.
Das erfindungsgemäß bevorzugte Ubichinonderivat ist das Coenzym Q 10.
Ein erfindungsgemäß bevorzugte Carnitinderivat ist das Acetylcarnitin.
Erfindungsgemäß vorteilhaft ist der Zusatz hydrophiler Vitamine, insbesondere Ascobinsäure, Folsäure, Liponsäure, Pyridoxin und Niacinamid.
Erfindungsgemäß vorteilhaft ist der Zusatz von mindestens einer Cyclodextrinspezies und/oder dessen Derivate.
Unter Cyclodextrinspezies sind erfindungsgemäß zu verstehen die nativen Cyclodextrine $\alpha$-, $\beta$- und $\gamma$-Cyclodextrin. Unter die Derivate dieser Spezies fallen alle ganz oder teilweise an den Hydroxylgruppen veretherten und/oder veresterten und/oder anders derivatisierten $\alpha$-, $\beta$- und $\gamma$-Cyclodextrine, insbesondere aber nicht ausschließlich das Hydroxypropyl-β-Cyclodextrin sowie das Methyl-ß-Cyclodextrin.
Erfindungsgemäß ist dabei eine Konzentration von 0,001 bis 5% und insbesondere von 0.1 bis 2 Gewichts-% bezogen auf das Gesamtgewicht der Zubereitung an mindestens einer Cyclodextrinspezies und/oder dessen Derivaten vorteilhaft.

Die erfindungsgemäß besonders bevorzugte Cyclodextrinspezies ist dabei γ-Cyclodextrin.

Erfindungsgemäß vorteilhaft ist die Verwendung von Cyclodextrinspezies und/oder dessen Derivaten zur Erhöhung der Löslichkeit und biologischen Verfügbarkeit von Retinoiden, Biotin und Ubichinon und/oder dessen Derivaten.

Weiterhin ist die Verwendung von Cyclodextrinspezies und/oder dessen Derivaten zur Verbesserung der biologischen Wirksamkeit von Retinoiden, Biotin und Ubichinon und/oder dessen Derivaten erfindungsgemäß vorteilhaft.

Die erfindungsgemäßen kosmetischen und/oder dermatologischen Zubereitungen finden Verwendung zur Behandlung und/oder Prophylaxe der Symptome der intrisischen und /oder extrinsischen Hautalterung, einschließlich der trockenen Altershaut, zur Reduktion von Fältchen und Falten der Haut und/oder zur Verbesserung der Elastizität der Haut sowie zur Behandlung und/oder Prophylaxe der schädlichen Auswirkungen ultravioletter Strahlung auf die Haut und zur Verbesserung des Lipidgehaltes der Haut.

Die erfindungsgemäßen Wirkstoffkombinationen lassen sich problemlos üblichen kosmetischen Zubereitungen, vorteilhaft Lichtschutzzubereitungen, aber auch anderen Zubereitungen, beispielsweise pharmazeutischen Zubereitungen einverleiben.

Kosmetische Zubereitungen enthalten in der Regel eine Vielzahl an Hilfs- und Wirkstoffen, die auch in den erfindungsgemäßen Zubereitungen vorteilhafter Weise eingesetzt werden können.

Erfindungsgemäß können in den Zubereitungen, welche die erfindungsgemäßen Wirkstoffkombinationen enthalten, die üblichen Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D, L-Carnosin, D-Carnosin, L-Carnosin und deren Derivate (z.B. Anserin), Carotinoide, Carotine (z.B. α-Carotin, β-Carotin, Lycopin) und deren Derivate, Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z. B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 10 Gew.-%, besonders bevorzugt 0,025 - 2.0 Gew.-%, insbesondere 0.05 - 1.0 Gew.-%, bezogen auf das Gesamtgewicht der Zubereitung.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, dass der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche kosmetische und dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch, einer Mousse-Creme aus einem Aerosolbehälter sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wässrige Systeme bzw. Tensidzubereitungen zur Reinigung der Haut und der Haare einzufügen.

Es ist dem Fachmanne natürlich bekannt, dass anspruchsvolle kosmetische Zusammensetzungen zumeist nicht ohne die üblichen Hilfs- und Zusatzstoffe denkbar sind. Die erfindungsgemäßen kosmetischen Zubereitungen können daher kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, weitere Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy)-1,3,5-triazin.

Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion.
Weiterhin vorteilhafte UVA-Filter entstammen der Gruppe der Triazine, so z.B. das 2,4-Bis-{[4-(2-Ethyl-hexyloxy)-2-hydroxy]-phenyl}-6-(4-methoxyphenyl)-1,3,5-triazin (Handelsbezeichnung Tinosorb® S) sowie der Gruppe der Triazole, wie z.B. das 2,2'-Methylen-bis- [6-2H-benzotriazol-2yl]-4-(1,1,3,3-tetramethylbutyl)phenol) (Handelsbezeichnung Tinosorb® M). Ein vorteilhafter wasserlöslicher UVA-Filter stellt das 2'-bis-(1,4-Phenylen)-1H-benzimidazol-4,6-disulfonsäure-Natriumsalz dar (Handelsbezeichnung Neo Heliopan AP®).

Es können die für die UVB-Kombination verwendeten Mengen eingesetzt werden.

Erfindungsgemäße kosmetische und dermatologische Zubereitungen enthalten vorteilhaft außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$), Zirkoniums ($ZrO_2$), Siliziums ($SiO_2$), Mangans (z.B. $MnO$), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Rektion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Erfindungsgemäße Zubereitungen können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

$RNH_2^+CH_2CH_2COOH\ X^-$ (bei pH=2) $X^-$ = beliebiges Anion, z.B. $Cl^-$
$RNH_2^+CH_2CH_2COO^-$ (bei pH=7)
$RNHCH_2CH_2COO^-\ B^+$ (bei pH=12) $B^+$ = beliebiges Kation, z.B. $Na^+$

Typisch für nicht-ionische Tenside sind Polyether-Ketten. Nicht-ionische Tenside bilden in wässrigem Medium keine Ionen.

A. Anionische Tenside

Vorteilhaft zu verwendende anionische Tenside sind
Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,

3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. AcylLactylate, lauroyllactylat, Caproyllactylat
6. Alaninate

Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamid Carboxylat,

Phosphorsäureester und Salze, wie beispielsweise DEA-Oleth-10-Phosphat und Dilaureth-4 Phosphat, Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat

sowie
Schwefelsäureester, wie

1. Alkylethersulfat, beispielsweise Natrium-, Ammonium-, Magnesium-, MIPA-, TIPA- Laurethsulfat, Natriummyrethsulfat und Natrium $C_{12-13}$ Parethsulfat,
2. Alkylsulfate, beispielsweise Natrium-, Ammonium- und TEA- Laurylsulfat.

B. Kationische Tenside

Vorteilhaft zu verwendende kationische Tenside sind

1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

2. N-Alkylaminosäuren, beispielsweise Aminopropylalkylglutamid, Alkylaminopropionsäure, Natriumalkylimidodipropionat und Lauroamphocarboxyglycinat..

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.
Die oberflächenaktive Substanz kann in einer Konzentration zwischen 1 und 30 Gew.-% in den erfindungsgemäßen Zubereitungen vorliegen, bezogen auf das Gesamtgewicht der Zubereitungen.
Die Lipidphase der erfindungsgemäßen kosmetischen oder dermatologischen Emulsionen kann vorteilhaft gewählt werden aus folgender Substanzgruppe:

- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopro pyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.
Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.
Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.
Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether, Dicaprylylcarbonat.
Besonders vorteilhaft sind Mischungen aus $C_{12-15}$-Alkylbenzoat und 2-Ethylhexylisostearat, Mischungen aus

$C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an zyklischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

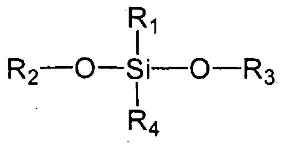

Als erfindungsgemäß vorteilhaft einzusetzenden linearen Silicone mit mehreren Siloxyleinheiten werden im allgemeinen durch Strukturelemente charakterisiert wie folgt:

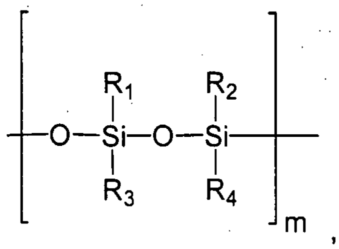

wobei die Siliziumatome mit gleichen oder unterschiedlichen Alkylresten und/oder Arylresten substituiert werden können, welche hier verallgemeinernd durch die Reste $R_1$ - $R_4$ dargestellt sind (will sagen, dass die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende zyklische Silicone werden im allgemeinen durch Strukturelemente charakterisiert, wie folgt

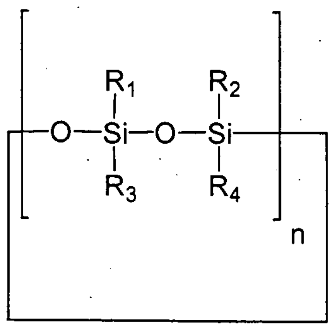

wobei die Siliziumatome mit gleichen oder unterschiedlichen Alkylresten und/oder Arylresten substituiert werden können, welche hier verallgemeinernd durch die Reste $R_1$ - $R_4$ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Zyklus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan), Cetyldimethicon, Behenoxydimethicon.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle- ähnlicher Konstitution wie der vorstehend bezeichneten Verbindungen

zu wählen, deren organische Seitenketten derivatisiert, beispielsweise polyethoxyliert und/oder polypropoxyliert sind. Dazu zählen beispielsweise Polysiloxanpolyalkyl-polyether-copolymere wie das Cetyl-Dimethicon-Copolyol, das (Cetyl-Dimethicon-Copolyol (und) Polyglyceryl-4-Isostearat (und) Hexyllaurat)

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliziumdioxid, Aluminiumsilikate.

Erfindungsgemäße als Emulsionen vorliegenden Zubereitungen enthalten insbesondere vorteilhaft ein oder mehrere Hydrokolloide. Diese Hydrokolloide können vorteilhaft gewählt werden aus der Gruppe der Gummen, Polysaccharide, Cellulosederivate, Schichtsilikate, Polyacrylate und/oder anderen Polymeren.

Erfindungsgemäße als Hydrogele vorliegenden Zubereitungen enthalten ein oder mehrere Hydrokolloide. Diese Hydrokolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Goodrich (Carbopol 980, 981, 1382, 5984, 2984, ETD 2001, ETD 2020, ETD 2050, Ultrez 10 oder Pemulen TR1 & TR2).

Unter den Polymeren befinden sich z.B. Polyacrylamide (Seppigel 305), Polyvinylalkohole, PVP, PVP / VA Copolymere, Polyglycole.

Erfindungsgemäße als Emulsionen vorliegenden Zubereitungen enthalten einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich

a) Partialfettsäureester und Fettsäureester mehrwertiger Alkohole und deren ethoxylierte Derivate (z. B. Glycerylmonostearate, Sorbitanstearate, Glycerylstearylcitrate, Sucrosestearate)
b) ethoxylierte Fettalkohole und Fettsäuren
c) ethoxilierte Fettamine, Fettsäureamide, Fettsäurealkanolamide
d) Alkylphenolpolyglycolether (z.B. Triton X)
e) Zuckerderivate (Ester und/oder Ether von Glucose, Saccharose und anderen Zuckern; z.B. Alkylpolyglycoside wie Polyglyceryl-3-Methylglucose-Distearat, Methylglucosesesquistearat )

Unter den anionischen Emulgatoren befinden sich

a) Seifen (z. B. Natriumstearat)
b) Fettalkoholsulfate
c) Mono-, Di- und Trialkylphosphosäureester und deren Ethoxylate

Unter den kationischen Emulgatoren befinden sich

a) quaternäre Ammoniumverbindungen mit einem langkettigen aliphatischen Rest z.B. Distearyldimonium Chloride

Unter den amphoteren Emulgatoren befinden sich

a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:

- der Fettalkoholethoxylate
- der ethoxylierten Wollwachsalkohole,
- der Polyethylenglycolether der allgemeinen Formel

$$R\text{-}O\text{-}(\text{-}CH_2\text{-}CH_2\text{-}O\text{-})_n\ R',$$

- der Fettsäureethoxylate der allgemeinen Formel

$$R\text{-}COO\text{-}(\text{-}CH_2\text{-}CH_2\text{-}O\text{-})_n\ \text{-}H,$$

- der veretherten Fettsäureethoxylate der allgemeinen Formel

$$R\text{-}COO\text{-}(\text{-}CH_2\text{-}CH_2\text{-}O\text{-})_n\ \text{-}R',$$

- der veresterten Fettsäureethoxylate der allgemeinen Formel

$$R\text{-}COO\text{-}(\text{-}CH_2\text{-}CH_2\text{-}O\text{-})_n\ \text{-}C(O)\text{-}R',$$

- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

$$R\text{-}O\text{-}(\text{-}CH_2\text{-}CH_2\text{-}O\text{-})_n\text{-}CH_2\text{-}COOH$$

nd n eine Zahl von 5 bis 30 darstellen,

- der Polyoxyethylensorbitolfettsäureester,
- der Alkylethersulfate der allgemeinen Formel

$$R\text{-}O\text{-}(\text{-}CH_2\text{-}CH_2\text{-}O\text{-})_n\text{-}SO_3\text{-}H$$

- der Fettalkoholpropoxylate der allgemeinen Formel

$$R\text{-}O\text{-}(\text{-}CH_2\text{-}CH(CH_3)\text{-}O\text{-})_n\text{-}H,$$

- der Polypropylenglycolether der allgemeinen Formel

$$R\text{-}O\text{-}(\text{-}CH_2\text{-}CH(CH_3)\text{-}O\text{-})_n\text{-}R'.$$

- der propoxylierten Wollwachsalkohole,

- der veretherten Fettsäurepropoxylate

$$R\text{-}COO\text{-}(\text{-}CH_2\text{-}CH(CH_3)\text{-}O\text{-})_n\text{-}R',$$

- der veresterten Fettsäurepropoxylate der allgemeinen Formel

$$R\text{-}COO\text{-}(\text{-}CH_2\text{-}CH(CH_3)\text{-}O\text{-})_n\text{-}C(O)\text{-}R',$$

- der Fettsäurepropoxylate der allgemeinen Formel

$$R\text{-}COO\text{-}(\text{-}CH_2\text{-}CH(CH_3)\text{-}O\text{-})_n\text{-}H,$$

- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

$$R\text{-}O\text{=}(\text{-}CH_2\text{-}CH(CH_3)O\text{-})_n\text{-}CH_2\text{-}COOH$$

- der Alkylethersulfate bzw. die diesen Sulfaten zugrundeliegenden Säuren der allgemeinen Formel

$$R\text{-}O\text{-}(\text{-}CH_2\text{-}CH(CH_3)\text{-}O\text{-})_n\text{-}SO_3\text{-}H$$

- der Fettalkoholethoxylate/propoxylate der allgemeinen Formel

$$R\text{-}O\text{-}X_n\text{-}Y_m\text{-}H,$$

- der Polypropylenglycolether der allgemeinen Formel

$$R\text{-}O\text{-}X_n\text{-}Y_m\text{-}R',$$

- der veretherten Fettsäurepropoxylate der allgemeinen Formel

$$R\text{-}COO\text{-}X_n\text{-}Y_m\text{-}R',$$

- der Fettsäureethoxylate/propoxylate der allgemeinen Formel

$$R\text{-}COO\text{-}X_nY_m\ H,$$

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.
Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol (20)isostearylether (Isosteareth-20),

Polyethylenglycol(13)cetylether (Ceteth-13), Polyethylenglycol(14)cetylether (Ceteth-14), Polyethylenglycol (15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17) isocetylether (Isoceteth-17), Polyethylenglycol(18)isocetylether (Isoceteth-18), Polyethylenglycol(19)isocetylether (Isoceteth-19); Polyethylenglycol(20)isocetylether (Isoceteth-20),

Polyethylenglycol(12)oleylether (Oleth-12), Polyethylenglycol(13)oleylether (Oleth-13), Polyethylenglycol (14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)-cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol (23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15) oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

## Beispiele 1-10: O/W-Cremes

| **Beispiel Nummer** | **1** | **2** | **3** | **4** | **5** |
|---|---|---|---|---|---|
| Glycerylstearatcitrat | 2 | | | 2 | |
| Glycerylsterat, selbstemulgierend | | 5 | 3 | | 2 |
| PEG-40-Stearat | | | 1 | | 1 |
| Myristylmyristat | 1 | | | | 1 |

| | | | | | |
|---|---|---|---|---|---|
| Behenylalkohol | | | | | |
| Stearylalkohol | 2 | 1 | | | |
| Cetearylalkohol | | | | 4 | 2 |
| Cetylalkohol | 1 | | 3 | | |
| Hydrierte Kokosfettglyceride (Hydrogenated Coco Glycerides) | 2 | | | | |
| Sheabutter | | 2 | | | 2 |
| C12-15 Alkylbenzoat | | 3 | 2 | | 3 |
| Butylenglycol Dicaprylat/Dicaprat | 1 | | | 1 | |
| Caprylsäure/Caprinsäure Triglycerid | | 1 | 1 | 2 | 2 |
| Ethylhexylkokosfettsäureester | 3 | | | | 1 |
| Octyldodecanol | | | 1 | | |
| Mineralöl | | 1 | | | |
| Vaseline | 2 | | 1 | | 2 |

| Beispiel Nummer | 1 | 2 | 3 | 4 | 5 |
|---|---|---|---|---|---|
| Octamethyltetrasiloxan (Cyclomethicon) | 4 | 1 | 4 | 3 | 5 |
| Dimethylpolysiloxan (Dimethicon) | | | 1 | 1 | |
| Dicaprylylether | 1 | 4 | 2 | | |
| Dicarprylylcarbonat | | | | 3 | |
| $TiO_2$ | | | 1 | 0,5 | 1 |
| Ethylhexylmethoxycinnamat | 3 | 3 | 5 | | 2 |
| Ethylhexyltriazon | | 2 | | | |
| Ethylhexylcyanodiphenyl-acrylat (Octocrylen) | | | | 5 | |
| Butylmethoxydibenzoyl-methan | | | | 1 | |
| Bis-Ethylhexyloxyphenol-methoxyphenyltriazine | 1 | 0,5 | | | |
| Ethylhexylsalicylat | | | 1 | | |
| Ubichinon (Q10) | 0,05 | 0,1 | 0,02 | 0,01 | 0,03 |
| Biotin | 0,2 | 0,05 | 0,04 | 0,01 | 0,04 |
| Retinol | 0,05 | | 0,03 | | |
| Retinylpalmitat | | 0,2 | | 0,1 | 0,3 |
| Acetylcarnitin | | | | 0,3 | |
| Tocopherylacetat | | | 1 | | |
| Citronensäure, Natriumsalz | | 0.1 | | | |
| Natriumascorbylpalmitat | 0,1 | | | | 0,1 |
| γ-Cyclodextrin | 2,0 | | 0.5 | 1,0 | 0.8 |

| **Beispiel Nummer** | **1** | **2** | **3** | **4** | **5** |
|---|---|---|---|---|---|
| Trinatrium EDTA | | 0.1 | | 0,2 | |
| Iminodisuccinat, Natriumsalz | 0,2 | | 0,1 | | 0,1 |
| Phenoxyethanol | 0,3 | | 0,3 | 0,2 | 0,2 |
| p-Hydroxybenzoesäurealkylester (Paraben) | 0,6 | 0,3 | 0,2 | 0,3 | 0,3 |
| Hexamidindiisethionat | | 0,04 | | | |
| Diazolidinylharnstoff | 0,25 | | 0,1 | 0,2 | 0,1 |
| 1,3-Dimethylol-5,5-dimethylhydantoin (DMDM Hydantoin) | | 0,2 | | | |
| Iodopropynylbutylcarbamat | | 0,1 | | | |
| Ethanol denaturiert | | 2 | | | |
| Xanthan Gummi | 0,1 | | | | |
| Polyacrylsäure (Carbomer) | 0,05 | | 0,1 | | 0,1 |
| Polyacrylamid | | 0,2 | | | |
| Glycerin | 10 | 6 | 6 | 7,5 | 8 |
| Butylenglycol | 2 | 1 | | | |
| wasser- und/oder öllösliche Farbstoffe | 0,05 | | | | |
| Füllstoffe/ Additive (Distärkephosphat, $SiO_2$, BHT, Talkum, Aluminiumstearat) | 0,1 | 1 | 0,2 | 0,5 | 0,05 |
| Parfüm | q.s. | q.s. | q.s. | q.s. | q.s. |
| Wasser | Ad 100 | Ad 100 | Ad 100 | Ad 100 | Ad 100 |

| **Beispiel Nummer** | **6** | **7** | **8** | **9** | **10** |
|---|---|---|---|---|---|
| Glycerylsterat, selbstemulgierend | 2,5 | | | | |
| PEG-40-Stearat | 1 | | | | |
| Polyglyceryl-3-Methylglucosedistearat | | 3 | | | |
| Sorbitanstearat | | 1 | | | |
| Polyethylenglycol(21)stearylether (Steareth-21) | | | 2 | | |
| Polyethylenglycol(2)stearylether (Steareth-2) | | | 1 | | |
| Cetearylglucosid | | | | 2 | |
| Stearinsäure | | | | | 2,5 |
| Myristylmyristat | | | | 1 | |
| Behenylalkohol | | 1 | | | 2 |
| Stearylalkohol | | | | 5 | |
| Cetearylalkohol | 3 | | 2 | | 1 |
| Cetylalkohol | | 1 | | | |
| Hydrierte Kokosfettglyceride (Hydrogenated Coco Glycerides) | 1 | | | | 1 |
| Sheabutter | 2 | | | | |
| C12-15 Alkylbenzoat | 4 | 2 | 5 | 2 | |
| Butylenglycol Dicaprylat/Dicaprat | | | | | 2 |
| Caprylsäure/Caprinsäure Triglycerid | 1 | 1 | | 3 | |
| Hydriertes Polydecen | | | | 1 | |
| Ethylhexylkokosfettsäureester | | | | | 2 |
| Octyldodecanol | | | 1 | | 1 |
| Mineralöl | | | 1 | | |

| Beispiel Nummer | 6 | 7 | 8 | 9 | 10 |
|---|---|---|---|---|---|
| Vaseline | 1 | | | | |
| Octamethyltetrasiloxan (Cyclomethicon) | 4 | 3 | 2 | | 2 |
| Dimethylpolysiloxan (Dimethicon) | | | | | 1 |
| Dicaprylylether | | | 2 | | |
| Dicarprylylcarbonat | | 2 | | 3 | 4 |
| Polydecen | | | | 4 | |
| $TiO_2$ | 1 | | 1 | | 1 |
| Ethylhexylmethoxycinnamat | | 3 | | 5 | |
| Ethylhexylcyanodiphenyl-acrylat (Octocrylen) | 5 | | | | 3 |
| Ethylhexyltriazon | | 2 | 4 | | |
| Phenylbenzimidazol-sulfonsäure | 0,5 | | | | 1 |
| Bis-Ethylhexyloxyphenol-methoxyphenyltriazine | | 1 | | 2 | 0,5 |
| Ubichinon (Q10) | 0,03 | 0,1 | 0,02 | 0,3 | 0,1 |
| Biotin | | 0,02 | | 0,10 | 0,5 |
| Retinol | | 0,04 | | | |
| Retinylpalmitat | 0,1 | | 0,5 | 0,2 | 0,3 |
| Tocopherylacetat | | 0,3 | | | 0,5 |
| Carnitin | 1 | | | | |
| Acetylcarnitin | | | 0,5 | | |
| Ascorbinsäure | | | 0,1 | | |
| β-Cyclodextrin | | | 0,3 | | |
| γ-Cyclodextrin | | 1,0 | 0,3 | 0,8 | 1 |

| Beispiel Nummer | 6 | 7 | 8 | 9 | 10 |
|---|---|---|---|---|---|
| Lactoferrin | | | | | 0,05 |
| Trinatrium EDTA | | | 0,2 | 0,1 | |
| Iminodisuccinat | 0,2 | 0,2 | | | 0,1 |
| Phenoxyethanol | 0,5 | 0,4 | 0,5 | | 0,3 |
| p-Hydroxybenzoesäurealkylester (Paraben) | 0,1 | | | 0,4 | 0,6 |
| Hexamidindiisethionat | | | 0,1 | | |
| Diazolidinylharnstoff | 0,2 | 0,2 | | 0,1 | |
| Iodopropynylbutylcarbamat | | | 0,25 | | |
| Ethanol denaturiert | | 8 | | | 3 |
| 2-Ethylhexylglycerinether (Octoxyglycerin) | | | | 0,4 | |
| Xanthan Gummi | | 0,1 | | | |
| Polyacrylsäure (Carbomer) | 0,2 | | 0,1 | | 0,1 |
| Polyacrylamid | | 0,2 | | | |
| Glycerin | 10 | 5 | 6 | 4 | 7 |
| Butylenglycol | | | | 2 | |
| wasser- und/oder öllösliche Farbstoffe | | | | | 0,1 |
| Additive (Distärkephosphat, $SiO_2$, Talkum, BHT Aluminiumstearat) | 0.03 | 0,1 | 0,05 | 3 | 1 |
| Parfüm | q.s. | q.s. | q.s. | q.s. | q.s. |
| Wasser | Ad 100 | Ad 100 | Ad 100 | Ad 100 | Ad 100 |

| **Beispiel 11: W/O-Creme** | |
|---|---|
| Polyglyceryl-3-Diisostearat | 5,0 |
| Polyglyceryl-2-Dipolyhydroxystearat | 2,5 |
| Cetearylalkohol | 2 |
| Cetylalkohol | 2 |
| C12-15 Alkylbenzoat | 8 |

(fortgesetzt)

| Beispiel 11: W/O-Creme | |
|---|---|
| Caprylsäure/Caprinsäure Triglycerid | 6 |
| Octyldodecanol | 5 |
| Octamethyltetrasiloxan (Cyclomethicon) | 2 |
| Milchsäure | 5 |
| Citronensäure, Natriumsalz | 0,5 |
| Butylmethoxydibenzoylmethan | 0,5 |
| Ethylhexyltriazon | 1 |
| Ethylhexylmethoxycinnamat | 5 |
| Ubichinon (Q10) | 0,05 |
| Biotin | 0.04 |
| Retinylpalmitat | 0,1 |
| Phenoxyethanol | 0,1 |
| p-Hydroxybenzoesäurealkyleste (Paraben) | 0,1 |
| Glycerin | 7.5 |
| β-Cyclodextrin | 0.2 |
| Füllstoffe (Distärkephosphat, $SiO_2$, Talkum, Aluminiumstearat) | 0,2 |
| Parfüm | q.s. |
| Wasser | Ad 100 |

| Beispiel 12: Hydrodispersion/Gelcreme | |
|---|---|
| Cetylalkohol | 2 |
| Sheabutter | 1 |
| Caprylsäure/Caprinsäure Triglycerid | 2 |
| Octyldodecanol | 1 |
| Octamethyltetrasiloxan (Cyclomethicon) | 5 |
| Dimethylpolysiloxan (Dimethicon) | 1 |
| Polydecen | 2 |
| Ethylhexylmethoxycinnamat | 3 |
| Bis-Ethylhexyloxyphenol-methoxyphenyltriazin | 0,5 |
| Ubichinon (Q10) | 0,02 |
| Biotin | 0.03 |
| Retinylpalmitat | 0,05 |
| Iminodisuccinat | 0,2 |
| Phenoxyethanol | 0,3 |
| p-Hydroxybenzoesäurealkyleste (Paraben) | 0,4 |
| Vernetztes Alkylacrylat (Alkylacrylate Cosspolymer) | 0,2 |
| Glycerin | 5 |

(fortgesetzt)

| Beispiel 12: Hydrodispersion/Gelcreme | |
|---|---|
| Parfüm | q.s. |
| Wasser | Ad 100 |

| Beispielrezepturen Foundations | | | | |
|---|---|---|---|---|
| **Inhaltsstoffe** | **1** | **2** | **3** | **4** |
| PEG-30-stearat | 1,5 | | | |
| Gylcerylstearat | 0,5 | | | 2 |
| Ceteareth-20 | 1 | | | |
| Polyglyceryl-3 Methylglucose stearat | | | 3,5 | |
| Sorbinsäurestearat | | | 1,5 | |
| Stearinsäure | 2 | | | 1,8 |
| Glycerylstearatcitrat. | | 3,5 | | |
| Cetylalkohol | 0,5 | 0,75 | 1,0 | |
| Lecithin | | | | 0,5 |
| Veegum K = Mg-Al-Silicate. | 0,8 | | | 1 |
| Xanthan Gum | 0,2 | | | 0,3 |
| Carbomer | | 0,2 | | |
| Hydroxyethylcellulose | 0,2 | | 0,1 | |
| Caprylsäure/ Caprinsäure Triglycerid | | 2 | 3 | 2 |
| Dicaprylylether | | 2 | 3 | 3 |
| Octyldodecanol | | | 3 | |
| Dimethicon | 3 | | 3 | 3 |
| Cyclomethicon | | 4 | 3 | 2 |
| C12-C15 Alkyl Benzoate | 2 | | | |
| Cetearyloctanoat | 2 | | | |
| Squalan | 1 | | | 6 |
| Isopropylpalmitat | 1 | | | 2 |
| PPG -15 Stearylether | 2 | 3 | | |
| Hydrierete Kokos-Glyceride | 2 | | | |
| Stearyldimethicon | 9 | | | |
| Acetyliertes Lanolinöl | 2 | 0,2 | | |
| Ethylhexylmethoxycinnamat | 2 | 2 | | 2 |
| $TiO_2$ | | 2 | | 2 |
| Ethylhexyltriazon | 2 | | | 1 |
| Bis-Ethylhexyloxyphenolmethoxyphenyltriazin | | 1 | 2 | |
| Ubichinon (Q10) | 0,1 | 0,05 | 0,02 | 0,025 |
| Retinylpalmitat | 0,02 | 0,05 | 0,1 | 0,1 |

(fortgesetzt)

| Beispielrezepturen Foundations | | | | |
|---|---|---|---|---|
| **Inhaltsstoffe** | **1** | **2** | **3** | **4** |
| Biotin | 0,01 | 0,025 | 0,02 | 0,05 |
| γ-Cyclodextrin | | | 0.75 | 0.05 |
| Silikon | | 0,8 | | |
| Nylon-12 | | | 5 | |
| Lauroyllysin | 0,5 | | 0,5 | |
| Kaolin | 0,5 | | 1 | 2 |
| Natrium Stärke Octenylsuccinat | | 1 | | 1,5 |
| Eisenoxide | 1,2 | 2,4 | 2,6 | 3 |
| Titandioxid | 3,8 | 5,6 | 4,5 | 6,5 |
| Interferenz Pigmente | 0,2 | | | 0,5 |
| Pigment Low Lustre | 0,2 | | | 0,2 |
| ZnO | | 1 | | |
| Polymethylsilsesquioxan (Tospearl 2000B) | | 2 | | |
| EDTA | 0,1 | 0,6 | 1 | 1 |
| Glycerin | 2 | 5 | 5 | 10 |
| Phenoxyethanol und Paraben (Phenonip) | 1 | 0,5 | | 1 |
| Imidazonidinylharnstoff | 0,3 | 0,3 | | 0,25 |
| Neutralisationsmittel | q.s. | q.s. | q.s. | q.s. |
| Parfum, Antioxidantien | q.s. | q.s. | q.s. | q.s. |
| Wasser | ad 100 | ad 100 | ad 100 | ad 100 |

**Patentansprüche**

**1.** Kosmetische und/oder dermatologische Zubereitungen enthaltend

a) mindestens ein Retinoid,
b) mindestens ein Ubichinon und/oder ein Derivat davon und
c) mindestens einen stickstoffhaltigen Wirkstoff aus der Gruppe Biotin, Carnitin und/oder deren Derivate

neben anderen kosmetischen Hilfs-, Wirk- und Zusatzstoffen.

**2.** Kosmetische und/oder dermatologische Zubereitungen nach Anspruch 1 enthaltend

a) Retinoid in einer Konzentration von 0,001 bis 1,0 Gewichts-%
b) Ubichinon in einer Konzentration von 0,001 bis 1,0 Gewichts-%
c) stickstoffhaltigen Wirkstoff aus der Gruppe Biotin, Carnitin und/oder Derivate davon in einer Konzentration von 0,01 bis 5,0 Gewichts-% jeweils bezogen auf das Gesamtgewicht der Zubereitung.

**3.** Kosmetische und/oder dermatologische Zubereitungen nach einem der Ansprüche 1 oder 2, worin Retinol das bevorzugte Retinoid ist.

**4.** Kosmetische und/oder dermatologische Zubereitungen nach einem der Ansprüche 1 bis 3, worin Coenzym Q 10 das bevorzugte Ubichinonderivat ist.

**5.** Kosmetische und/oder dermatologische Zubereitungen nach einem der Ansprüche 1 bis 4, worin Acetylcarnitin das bevorzugte Carnitinderivat ist.

**6.** Kosmetische und/oder dermatologische Zubereitungen nach einem der Ansprüche 1 bis 5, die hydrophile Vitamine enthalten.

**7.** Kosmetische und/oder dermatologische Zubereitungen nach einem der Ansprüche 1 bis 6 enthaltend mindestens eine Cyclodextrinspezies und/oder dessen Derivate.

**8.** Kosmetische und/oder dermatologische Zubereitungen nach einem der Ansprüche 1 bis 7 enthaltend mindestens eine Cyclodextrinspezies und/oder dessen Derivate in einer Konzentration von 0,001 bis 5 Gewichts-% bezogen auf das Gesamtgewicht der Zubereitung.

**9.** Kosmetische und/oder dermatologische Zubereitungen nach einem der Ansprüche 1 bis 7, worin $\gamma$-Cyclodextrin die bevorzugte Cyclodextrinspezies ist.

**10.** Verwendung von Cyclodextrinspezies und/oder dessen Derivaten zur Erhöhung der Löslichkeit und biologischen Wirksamkeit von Retinoiden, Biotin, Carnitin und/oder dessen Derivate und Ubichinon und/oder dessen Derivate in Form von kosmetischen und/oder dermatologischen Zubereitungen nach einem der Ansprüche 1 bis 9.

**11.** Verwendung kosmetischer und/oder dermatologischer Zubereitungen nach einem der Ansprüche 1 bis 9 zur Behandlung und/oder Prophylaxe der Symptome der intrisischen und /oder extrinsischen Hautalterung, einschließlich der trockenen Altershaut.

**12.** Verwendung kosmetischer und/oder dermatologischer Zubereitungen nach einem der Ansprüche 1 bis 9 zur Reduktion von Fältchen und Falten der Haut und / oder zur Verbesserung der Elastizität der Haut.

**13.** Verwendung kosmetischer und/oder dermatologischer Zubereitungen nach einem der Ansprüche 1 bis 9 zur Behandlung und/oder Prophylaxe der schädlichen Auswirkungen ultravioletter Strahlung auf die Haut.

**14.** Verwendung kosmetischer und/oder dermatologischer Zubereitungen nach einem der Ansprüche 1 bis 9 zur Verbesserung des Lipidgehaltes der Haut.

Europäisches Patentamt

# EUROPÄISCHER RECHERCHENBERICHT

Nummer der Anmeldung
EP 03 00 3098

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 6 048 886 A (NEIGUT STANLEY)<br>11. April 2000 (2000-04-11)<br>* Ansprüche 1,9,11 *<br>* Spalte 2, Zeile 15-27 *<br>* Spalte 4, Zeile 39-63 *<br>* Spalte 8, Zeile 10-30; Beispiel 3 *<br>--- | 1-6,<br>11-14 | A61K7/48 |
| A | US 4 371 673 A (PITHA JOSEF)<br>1. Februar 1983 (1983-02-01)<br>* Spalte 1, Zeile 9-22 *<br>* Spalte 2, Zeile 40-45 *<br>--- | 1-14 | |
| Y | DE 198 20 392 A (BEIERSDORF AG)<br>11. November 1999 (1999-11-11)<br>* Ansprüche 1,4,5,8,9 *<br>* Beispiele I-VII *<br>* Spalte 4, Zeile 8-16 *<br>* Spalte 6, Zeile 55-64 *<br>--- | 1-14 | |
| Y | DE 198 06 947 A (BEIERSDORF AG)<br>26. August 1999 (1999-08-26)<br>* Ansprüche 1-7 *<br>* Beispiele 1-9 *<br>* Spalte 1, Zeile 29 - Spalte 2, Zeile 3 *<br>--- | 1-14 | RECHERCHIERTE SACHGEBIETE (Int.Cl.7)<br>A61K |
| Y | EP 1 088 547 A (BEIERSDORF AG)<br>4. April 2001 (2001-04-04)<br>* Ansprüche 1-6 *<br>Beispiele<br>* Seite 7, Zeile 36 - Seite 8, Zeile 32 *<br>* Seite 9, Zeile 50-52 *<br>--- | 1-14 | |
| A | FR 2 706 301 A (CLARINS)<br>23. Dezember 1994 (1994-12-23)<br>* Ansprüche 5,6 *<br>* Seite 1, Zeile 1 - Zeile 2 *<br>---<br>-/-- | 1-6,<br>11-14 | |

Der vorliegende Recherchenbericht wurde für alle Patentansprüche erstellt

| Recherchenort | Abschlußdatum der Recherche | Prüfer |
|---|---|---|
| MÜNCHEN | 23. April 2003 | Grillenberger, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 03 00 3098

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | FR 2 647 015 A (CIRD)<br>23. November 1990 (1990-11-23)<br>* Ansprüche 1-11 *<br>* Beispiele I-IV *<br>--- | 1-14 | |
| A | US 4 727 064 A (PITHA JOSEF)<br>23. Februar 1988 (1988-02-23)<br>* Spalte 3; Tabelle 1 *<br>* Spalte 3, Zeile 48-58 *<br>* Spalte 4, Zeile 59-63 *<br>----- | 1-14 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| MÜNCHEN | 23. April 2003 | Grillenberger, S |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 03 00 3098

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-04-2003

| Im Recherchenbericht angeführtes Patentdokument | Datum der Veröffentlichung | Mitglied(er) der Patentfamilie | Datum der Veröffentlichung |
|---|---|---|---|
| US 6048886 A | 11-04-2000 | KEINE | |
| US 4371673 A | 01-02-1983 | CA 1175044 A1 | 25-09-1984 |
| | | DE 3172673 D1 | 21-11-1985 |
| | | EP 0056056 A1 | 21-07-1982 |
| | | WO 8200251 A1 | 04-02-1982 |
| DE 19820392 A | 11-11-1999 | DE 19820392 A1 | 11-11-1999 |
| | | AT 209481 T | 15-12-2001 |
| | | DE 59900473 D1 | 10-01-2002 |
| | | WO 9956719 A1 | 11-11-1999 |
| | | EP 1073412 A1 | 07-02-2001 |
| | | JP 2002513746 T | 14-05-2002 |
| | | US 2002182199 A1 | 05-12-2002 |
| DE 19806947 A | 26-08-1999 | DE 19806947 A1 | 26-08-1999 |
| EP 1088547 A | 04-04-2001 | DE 19941769 A1 | 08-03-2001 |
| | | EP 1088547 A2 | 04-04-2001 |
| | | US 6436414 B1 | 20-08-2002 |
| FR 2706301 A | 23-12-1994 | FR 2706294 A3 | 23-12-1994 |
| | | FR 2706301 A1 | 23-12-1994 |
| FR 2647015 A | 23-11-1990 | FR 2647015 A1 | 23-11-1990 |
| | | AU 5668790 A | 18-12-1990 |
| | | WO 9014082 A1 | 29-11-1990 |
| | | IT 1241141 B | 29-12-1993 |
| US 4727064 A | 23-02-1988 | US 4596795 A | 24-06-1986 |

EPO FORM P0461

Für nähere Einzelheiten zu diesem Anhang : siehe Amtsblatt des Europäischen Patentamts, Nr.12/82